(19) Europäisches Patentamt
European Patent Office
Office européen des brevets

(11) **EP 4 644 356 A1**

(12) **EUROPEAN PATENT APPLICATION**
published in accordance with Art. 153(4) EPC

(43) Date of publication:
**05.11.2025 Bulletin 2025/45**

(21) Application number: **23911962.1**

(22) Date of filing: **22.12.2023**

(51) International Patent Classification (IPC):
**C07C 29/151** (2006.01)  **C01B 3/38** (2006.01)
**C07B 61/00** (2006.01)  **C07C 31/04** (2006.01)

(52) Cooperative Patent Classification (CPC):
(C-Sets available)
**C07C 29/1518; C01B 3/38; C07B 61/00**   (Cont.)

(86) International application number:
**PCT/JP2023/046092**

(87) International publication number:
**WO 2024/143182 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.12.2022 JP 2022208964**

(71) Applicant: **MITSUBISHI GAS CHEMICAL
COMPANY, INC.**
**Chiyoda-ku
Tokyo 100-8324 (JP)**

(72) Inventors:
- **SHIBATA, Akihiro**
  **Tokyo 100-8324 (JP)**
- **ABE, Takanori**
  **Tokyo 100-8324 (JP)**
- **KAKIMI, Atsushi**
  **Tokyo 100-8324 (JP)**

(74) Representative: **Vossius & Partner
Patentanwälte Rechtsanwälte mbB
Siebertstrasse 3
81675 München (DE)**

(54) **METHANOL PRODUCTION METHOD AND METHANOL PRODUCTION DEVICE**

(57)    [Problem to be Solved]

To provide a method for producing methanol, in which the amount of carbon dioxide exhausted per the amount of methanol produced is small and the carbon yield is excellent.

[Solution]

A method for producing methanol including a step (A) of reforming a hydrocarbon-containing gas to obtain a reformed gas, a step (B) of mixing a hydrogen-containing gas with the hydrocarbon-containing gas and/or the reformed gas, a step (C) of reacting a portion of the reformed gas in the presence of a catalyst to obtain methanol and an unreacted gas, a step (D) of subjecting a residual portion of the reformed gas to a shift reaction to obtain a shift reaction gas, and a step (E) of separating carbon dioxide from the shift reaction gas to obtain a carbon dioxide-rich gas and a carbon dioxide separation unit off-gas, in which at least a portion of the carbon dioxide separation unit off-gas is used as a fuel of the step (A) and/or a boiler.

Figure 1

EP 4 644 356 A1

**EP 4 644 356 A1**

(52) Cooperative Patent Classification (CPC): (Cont.)

C-Sets
**C07C 29/1518, C07C 31/04**

**2**

**Description**

[Technical Field]

The present invention relates to a method for producing methanol and an apparatus for producing methanol.

[Background Art]

In conventional methods for industrially producing methanol using natural gas as a raw material, methanol is synthesized from a synthetic gas that is obtained by the steam reforming of the natural gas (main component: methane), which is a raw material. The steam reforming reaction is an endothermal reaction, there is a need to supply energy by combusting a large amount of a fuel to maintain a temperature suitable for the reaction (approximately 1000°C), and a large amount of carbon dioxide is exhausted. Examples of a method for reducing the amount of carbon dioxide exhausted include a method of recovering carbon dioxide from combustion exhaust gas (post combustion carbon dioxide recovery). The post combustion carbon dioxide recovery is an established technique, and there are also examples where the post combustion carbon dioxide recovery is applied to a commercial plant, but the carbon dioxide concentration in the combustion exhaust gas of a steam-reformed gas (reformed gas) is as low as 10 mol% or lower, which makes equipment or energy necessary to recover carbon dioxide relatively large.

Therefore, as a method for reducing carbon dioxide, methods in which carbon dioxide is separated and recovered from a reformed gas or an unreacted gas after methanol synthesis having a relatively high carbon dioxide concentration and then the low-carbon off-gas (main component: hydrogen) is used as a combustion fuel have been recently proposed as a mainstream method for reducing carbon dioxide in the production of methanol from natural gas.

All of such methods have hydrogen recovery equipment (PSA), carbon dioxide recovery equipment and a water shift reactor for increasing a hydrogen concentration and a carbon dioxide concentration as the configuration elements in many cases, and a variety of apparatus selections, disposition orders and gas compositions have been reported.

For example, Patent Literature 1 discloses, in the upstream of a line that supplies a synthetic gas flow to a methanol reaction apparatus, the supply of at least a portion of the synthetic gas flow to a water-gas shift reactor or the supply of a gas containing hydrogen obtained by the supply of the synthetic gas flow as a main component to a combustion heating apparatus or the like for the purpose of reducing the exhaustion of carbon dioxide.

Patent Literature 2 discloses a method including, in order to improve the amount of methanol produced or reduce energy consumption by reducing purge gas that is generated from a methanol synthesis apparatus, supplying the purge gas from the synthesis apparatus to a shift reactor, and furthermore supplying the same to a hydrogen separation apparatus, recycling recovered hydrogen, and exhausting off-gas to the outside of the system.

Patent Literature 3 discloses that, for the purpose of improving the stoichiometric value of a feed that is supplied to a methanol reactor, a portion of a reformed gas flow is passed through a shift reactor, then, the obtained gas is passed through a hydrogen separation apparatus, and hydrogen and the reformed gas flow are combined to adjust the M value of the reformed gas flow, whereby the consumption of natural gas that is used to produce synthetic gas can be significantly reduced.

Patent Literature 4 discloses a method including passing a reformed gas through a shift reactor to produce hydrogen and methanol at the same time.

[Citation List]

[Patent Literature]

[Patent Literature 1] EP3674261
[Patent Literature 2] US2017/0197894
[Patent Literature 3] EP3844135
[Patent Literature 4] US10160704

[Summary of Invention]

[Technical Problem]

However, in Patent Literature 1, it cannot be said that the gas flow that is supplied to the methanol synthesis reactor is sufficiently adjusted so as to become a gas composition suitable for methanol synthesis, which is probably because attention is paid to the reduction of carbon dioxide.

In Patent Literature 2, while it is disclosed that hydrogen is recycled by a shift reaction of the purge gas that is generated from the methanol synthesis apparatus, the gas composition cannot be said to be suitable for methanol synthesis, and thus it also cannot be said that the amount of carbon dioxide exhausted is sufficiently reduced in the entire system.

In Patent Literature 3, while the improvement in the stoichiometric value of the feed that is supplied to the methanol reactor and the like are described, it cannot be said that the amount of carbon dioxide exhausted is sufficiently reduced in the entire system.

In Patent Literature 4, while the reformed gas is passed through the shift reactor to produce hydrogen and methanol at the same time, it cannot be said that the gas composition is sufficiently adjusted to be suitable for methanol synthesis. In addition, it also cannot be said that the amount of carbon dioxide exhausted is sufficiently reduced in the entire system.

The present invention has been made in consideration of the above-described circumstances, and an objective of the present invention is to provide a method for producing methanol using natural gas as a raw material, in which the amount of carbon dioxide exhausted per the amount of methanol produced is small and the carbon yield is excellent.

[Solution to Problem]

As a result of intensive studies for achieving the above-described objective, the present inventors found that a method for producing methanol, in which the amount of carbon dioxide exhausted per the amount of methanol produced is small and the carbon yield is excellent, can be provided by a production method including predetermined steps in a predetermined order and completed the present invention. That is, the present invention is as described below.

[1] A method for producing methanol, comprising:

a step (A) of reforming a hydrocarbon-containing gas to obtain a reformed gas,
a step (B) of mixing a hydrogen-containing gas with the hydrocarbon-containing gas and/or the reformed gas,
a step (C) of reacting a portion of the reformed gas in the presence of a catalyst to obtain methanol and an unreacted gas,
a step (D) of subjecting a residual portion of the reformed gas to a shift reaction to obtain a shift reaction gas, and
a step (E) of separating carbon dioxide from the shift reaction gas to obtain a carbon dioxide-rich gas and a carbon dioxide separation unit off-gas,
in which at least a portion of the carbon dioxide separation unit off-gas is used as a fuel of the step (A) and/or a boiler.

[2] The method for producing methanol according to [1], further comprising
a step (F) of removing a sulfur content from the hydrocarbon-containing gas.
[3] The method for producing methanol according to [1] or [2], further comprising:
a step (G) of obtaining purified methanol from the methanol.
[4] The method for producing methanol according to any one of [1] to [3], in which a molar flow rate of the reformed gas that is used in the step (C) is 50 to 95 mol% based on a molar flow rate of reformed gas that is obtained in the step (A).
[5] The method for producing methanol according to any one of [1] to [4], in which steam that is generated in the step (C) is used in the step (G) and/or the step (E).
[6] The method for producing methanol according to any one of [1] to [5], in which steam recovered in the step (A) is used in the step (G) and/or the step (E).
[7] The method for producing methanol according to any one of [1] to [6], in which heat recovered in the step (A) is used as a heat source in at least any of the step (G) and the step (E).
[8] The method for producing methanol according to any one of [1] to [7], in which the step (D) is performed in the presence of a catalyst.
[9] The method for producing methanol according to any one of [1] to [8], in which a carbon monoxide concentration in the shift reaction gas is 0.01 to 1.0 mol%.
[10] The method for producing methanol according to any one of [1] to [9], in which a molar flow rate of carbon dioxide that is contained in the carbon dioxide-rich gas is 80 to 100 mol% based on a molar flow rate of carbon dioxide that is contained in the shift reaction gas being 100 mol%.
[11] The method for producing methanol according to any one of [1] to [10], in which at least a portion of the unreacted gas is supplied to a shift reaction unit and/or the boiler.

[12] The method for producing methanol according to any one of [1] to [11], further comprising

a step (H) of separating methane from the carbon dioxide separation unit off-gas to obtain a methane-rich gas and a methane separation unit off-gas,
in which the methane separation unit off-gas is supplied to a fuel of the step (A) and/or the boiler.

[13] The method for producing methanol according to any one of [1] to [12], in which the carbon dioxide separation unit off-gas and/or the methane separation unit off-gas is mixed with the hydrocarbon-containing gas and/or the reformed gas.

[14] The method for producing methanol according to any one of [1] to [13], in which a molar flow rate of methane that is contained in the methane-rich gas is 50 to 99 mol% based on a molar flow rate of methane that is contained in the carbon dioxide separation unit off-gas being 100 mol%.

[15] An apparatus for producing methanol, comprising:

a reforming unit;
a methanol synthesis unit;
a shift reaction unit; and
a carbon dioxide separation unit,
in which a carbon dioxide separation unit off-gas that is obtained from the carbon dioxide separation unit is used as a fuel of the reforming unit and/or a boiler.

[16] The apparatus for producing methanol according to [15], further comprising a desulfurization unit.

[17] The apparatus for producing methanol according to [15] or [16], further comprising a methane separation unit that separates methane from the carbon dioxide separation unit off-gas.

[Advantageous Effects of Invention]

According to the present invention, it is possible to provide a method for producing methanol in which the amount of carbon dioxide exhausted per the amount of methanol produced is small and the carbon yield is excellent.

[Brief Description of Drawings]

[Figure 1] Figure 1 is a schematic view showing an example of a production apparatus that is used in a method for producing methanol of the present invention.
[Figure 2] Figure 2 is a schematic view showing another example of the production apparatus that is used in the method for producing methanol of the present invention.
[Figure 3] Figure 3 is a schematic view showing still another example of the production apparatus that is used in the method for producing methanol of the present invention.
[Figure 4] Figure 4 is a schematic view showing far still another example of the production apparatus that is used in the method for producing methanol of the present invention.
[Figure 5] Figure 5 is a schematic view showing an example of a production apparatus that is used in a method for producing methanol that is equivalent to a comparative example.
[Figure 6] Figure 6 is a schematic view showing another example of the production apparatus that is used in the method for producing methanol that is equivalent to the comparative example.
[Figure 7] Figure 7 is a schematic view showing still another example of the production apparatus that is used in the method for producing methanol that is equivalent to the comparative example.
[Figure 8] Figure 8 is a schematic view showing far still another example of the production apparatus that is used in the method for producing methanol that is equivalent to the comparative example.
[Figure 9] Figure 9 is a schematic view showing even far still another example of the production apparatus that is used in the method for producing methanol that is equivalent to the comparative example.

[Description of Embodiments]

Hereinafter, an embodiment of the present invention (hereinafter, simply referred to as "the present embodiment") will be described in detail with reference to drawings as necessary, but the present invention is not limited to the following embodiment. The present invention can be modified in a variety of manners within the scope of the gist of the present invention. In the drawings, the same elements will be given the same reference sign and will not be described again. In addition, unless otherwise described, positional relationships of up, down, right and left are based on the positional relationships shown in the drawings. Furthermore, dimensional ratios in the drawings are not limited to the ratios shown in the drawings. In addition, unless particularly mentioned in the present embodiment, molar flow rates mean molar flow rates per unit time (kmol/h).

[Method for Producing Methanol]

A method for producing methanol of the present embodiment is a method for producing methanol, including:

a step (A) of reforming a hydrocarbon-containing gas to obtain a reformed gas,
a step (B) of mixing a hydrogen-containing gas with the hydrocarbon-containing gas and/or the reformed gas,
a step (C) of reacting a portion of the reformed gas in the presence of a catalyst to obtain methanol and an unreacted gas,
a step (D) of subjecting the residual portion of the reformed gas to a shift reaction to obtain a shift reaction gas, and
a step (E) of separating carbon dioxide from the shift reaction gas to obtain a carbon dioxide-rich gas and a carbon dioxide separation unit off-gas,
in which at least a portion of the carbon dioxide separation unit off-gas is used as a fuel of the step (A) and/or a boiler.

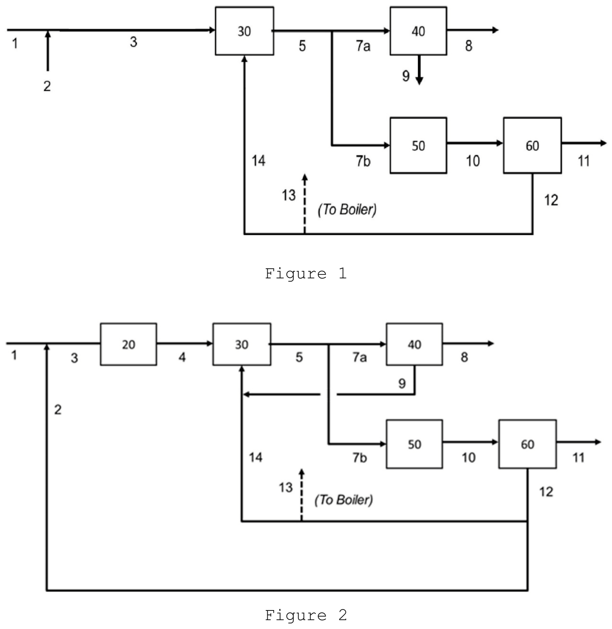

Hereinafter, the method for producing methanol of the present embodiment will be described using an apparatus for producing methanol of Figures 1 to 4. However, the method for producing methanol of the present embodiment is not limited to an embodiment in which the production apparatus of Figures 1 to 4 is used.

[Step (A)]

The step (A) in the method for producing methanol of the present embodiment is a step of reforming a hydrocarbon-containing gas 1 with a reforming unit 30 to obtain a reformed gas 5 as shown in Figures 1 to 4. Specifically, the step (A) is a step in which a hydrocarbon-containing gas and steam are reacted at a predetermined temperature to generate a reformed gas containing hydrogen ($H_2$), carbon monoxide (CO) and carbon dioxide ($CO_2$) as main components, which are used for the synthesis of methanol.

A reforming method is not particularly limited, and examples thereof include steam reforming (SMR), autothermal reforming (ATR), two-stage reforming (SMR + ATR) and partial oxidation. In addition, in a case where the hydrocarbon-containing gas is natural gas or naphtha, the method may include a pre-reforming step of reforming natural gas or naphtha at approximately 500°C by providing a pre-reformer upstream of the reforming unit to produce a methane-rich gas (for example, the $CH_4$ content rate is approximately 30 to 50 mol% while not particularly limited). In addition, from the viewpoint of obtaining a gas composition suitable for methanol synthesis, steam reforming is preferably used. In a case where steam reforming is used in the step (A), the step (A) is a step of reforming a gas mixture for reforming, in which a hydrocarbon-containing gas and steam are mixed together, to obtain a reformed gas.

As the reforming temperature in the step (A), a known conventional temperature can be used. The reforming temperature is not particularly limited and can be set to, for example, 750°C to 1000°C. In addition, a catalyst may be used in the step (A), and a known conventional catalyst can be used as such a catalyst. The catalyst is not particularly limited, and examples thereof include nickel-based catalysts.

The hydrocarbon-containing gas is not particularly limited, and examples thereof include purified gases and fossil fuel gases. The purified gases are not particularly limited, and examples thereof include methane, ethane, propane, butane and gas mixtures thereof. Examples of the fossil fuel gases include natural gas (NG) containing methane as a main component, liquefied petroleum gas (LPG) and naphtha. In a case where the hydrocarbon-containing gas is the fossil fuel gas, since a sulfur content is contained in the gas component, a gas that has undergone a desulfurization step, which will be described below, is preferably used as the hydrocarbon-containing gas.

Heat recovered in the step (A) may also be used as a heat source in at least any of a step (G) and the step (E).

[Step (B)]

The step (B) in the method for producing methanol of the present embodiment is a step of mixing the hydrocarbon-containing gas 1 and/or the reformed gas 5 and a hydrogen-containing gas 2. Figures 1 to 4 show aspects in which the hydrogen-containing gas 2 and the hydrocarbon-containing gas 1 are mixed upstream of the reforming unit 30. This makes it possible to make the gas composition suitable for methanol synthesis, and, in a case where the method for producing methanol of the present embodiment includes the desulfurization step, the step can be used to remove the sulfur content in a desulfurization unit 20.

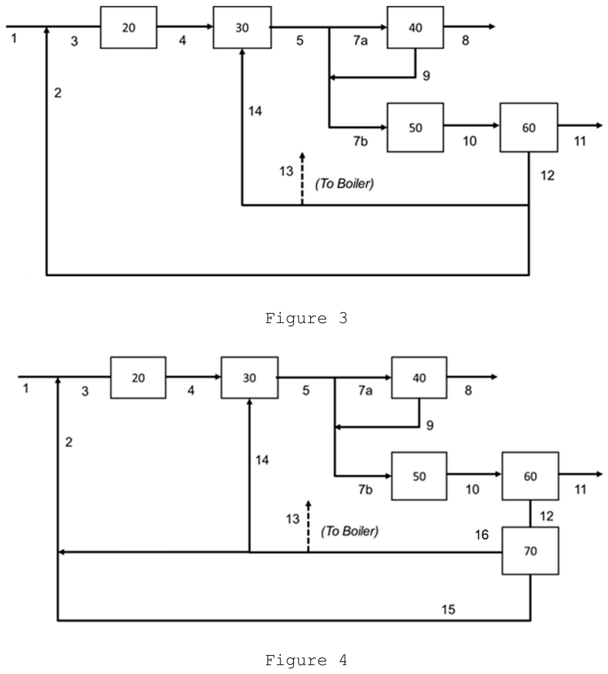

The hydrogen-containing gas is not particularly limited, and, for example, a carbon dioxide separation unit off-gas 12 may be used, a methane separation unit off-gas 16 shown in Figure 4 may be used, or hydrogen fabricated outside the system may be used.

In a case where hydrogen fabricated outside the system is used in the step (B), for example, hydrogen obtained using a renewable energy is preferably used from the viewpoint of decreasing the amount of carbon dioxide exhausted while not particularly limited. More specific examples thereof include by-product hydrogen from petroleum purification equipment, by-product hydrogen derived from a chemical process, blue hydrogen such as by-product hydrogen that has been jointly used with CCS, hydrogen having a gas composition adjusted by PSA or the like, water electrolysis hydrogen or seawater electrolysis hydrogen, hydrogen obtained by other electrolysis techniques and hydrogen that is obtained by steam reforming.

[Step (C)]

The step (C) in the method for producing methanol of the present embodiment is a step of supplying a portion 7a of the reformed gas to a methanol synthesis unit 40 and reacting the reformed gas in the presence of a catalyst to obtain methanol 8 and an unreacted gas 9 as shown in Figures 1 to 4. In addition, the unreacted gas 9 may be combined into a gas in a line 14 as shown in Figure 2 or may be combined into a gas in a line 7b as shown in Figures 3 and 4.

In addition, while not shown in Figures 1 to 4, in the step (C), it is possible to obtain methanol as a liquid phase and obtain an unreacted gas as a gas phase by cooling a reaction mixture that is obtained by the reaction and then separating the reaction mixture into gas and liquid. As a method for the gas-liquid separation, a known conventional method can be used. For example, a high-pressure separator can be used while not particularly limited.

The unreacted gas means a gas that is not used for the reaction in the methanol synthesis reaction. The unreacted gas is a gas mixture that can contain, as a composition thereof, hydrogen, carbon monoxide, carbon dioxide, methane, nitrogen and the like, which depends on the conditions for the methanol synthesis reaction. At least a portion of the unreacted gas is preferably supplied to a shift reaction unit and/or a boiler. In such a case, it is possible to further reduce the amount of carbon dioxide exhausted.

The portion of the reformed gas means not 100 mol% of the reformed gas. As the portion of the reformed gas, the molar flow rate of the reformed gas that is used in the step (C) is preferably 50 to 95 mol% and more preferably 60 to 90 mol% based on the molar flow rate of the reformed gas that is obtained in the step (A). When the molar flow rate of the reformed gas that is used in the step (C) is 95 mol% or less, there is a tendency that the reduction of the amount of carbon dioxide exhausted is superior, and, when the molar flow rate is 50 mol% or more, there is a tendency that the amount of methanol produced is superior.

The gas temperature at the inlet of the methanol synthesis unit 40 is set as appropriate depending on the kind or amount of the catalyst, the shape and reaction pressure of the reactor or the like and is preferably 170°C to 260°C, more preferably 170°C to 220°C and still more preferably 170°C to 200°C. When the gas temperature at the inlet is 170°C or higher, there is a tendency that the reactivity improves, and, when the gas temperature is 260°C or lower, there is a tendency that the device cost can be reduced.

The gas pressure at the inlet of the methanol synthesis unit 40 is preferably 4.9 to 14.7 MPaG, more preferably 5.0 to 11.0 MPaG and still more preferably 5.0 to 10.0 MPaG. When the gas pressure at the inlet is 4.9 MPaG or higher, there is a tendency that the reactivity improves, and, when the gas pressure is 14.7 MPaG or lower, there is a tendency that the production efficiency increases.

In the reformed gas 7a that is supplied to the methanol synthesis unit 40, the relationship (M value) among the mole percentages of CO, $CO_2$ and $H_2$ that is calculated by the following equation:

$$M \text{ value} = (H_2 \text{ mol\%})/(2 \times CO \text{ mol\%} + 3 \times CO_2 \text{ mol\%})$$

is preferably 1.3 to 5.0, more preferably 1.3 to 3.0, still more preferably 1.3 to 2.0 and particularly preferably 1.3 to 1.5. When the M value is 1.3 or more, there is a tendency that a by-product decreases, and, when the M value is 5.0 or less, there is a tendency that the carbon yield is excellent.

Here, the carbon yield means the rate of the molar flow rate of methanol that is generated in the methanol synthesis unit based on the total amount of the molar flow rate of carbon monoxide and the molar flow rate of carbon dioxide that are contained in the reformed gas that is supplied to the methanol synthesis unit.

The reaction temperature in the methanol synthesis unit 40 is preferably 200°C to 300°C, more preferably 200°C to 280°C and still more preferably 200°C to 270°C from the viewpoint of maintaining the reactivity, suppressing a by-product and protecting the catalyst.

The kind of the methanol synthesis unit 40 is not particularly limited, but is preferably, for example, a unit having a mechanism capable of controlling the reaction temperature. Specific examples thereof include a heat exchange-type reactor and a quench-type adiabatic reactor. The heat exchange-type reactor is not particularly limited, and examples thereof include a multi-tubular heat exchange-type reactor and a radial flow-type reactor.

In the case of using the multi-tubular heat exchange-type reactor, the reaction temperature is controlled by indirect heat exchange with pressurized boiling water, and saturated steam is obtained. The boiling water circulates through a steam drum and a shell side of the reactor, and the steam is recovered from the stream drum. The steam that is obtained from this synthesis system is preferably used as a heat source for the purification process of a methanol solution that is in the downstream of the synthesis process. In addition, the pressurized boiling water is preferably 220°C to 260°C.

In the case of employing the adiabatic reactor, one or more catalyst layers are provided inside, and, in a case where two or more layers are provided, a portion of a synthesis reactor supply gas is branched as a cooling gas of an interlayer and supplied as a quench gas, whereby the reaction temperature is controlled, steam is recovered by installing an evaporator as a heat-recovering apparatus in a reactor outlet gas, and, similarly, the steam may be used as a heat source for the purification process of a methanol solution in the downstream.

The catalyst that is used for the synthesis is preferably a methanol synthesis catalyst containing a copper atom and a zinc atom as essential components. Such a catalyst is reduced from an oxide state by a reducing gas, for example, hydrogen, carbon monoxide or a gas mixture thereof, whereby copper is activated to have a catalytic activity. The catalyst may contain, in addition to a copper atom and a zinc atom, an aluminum atom and/or a chromium atom as a main third component. The catalyst containing copper and zinc as essential components can be prepared by a known method. Such a catalyst can be prepared by, for example, the methods described in Japanese Patent Publication No. 51-44715, Japanese Patent No. 2695663, Japanese Patent Publication No. 6-35401, Japanese Patent Laid-Open No. 10-272361 and Japanese Patent Laid-Open No. 2001-205089.

A preferable catalyst is a methanol synthesis catalyst containing copper atoms and zinc atoms in an atomic ratio (copper/zinc) of 2.0 to 3.0 and containing an aluminum atom. Such a catalyst is not particularly limited, and examples thereof include a catalyst prepared by the method described in Japanese Patent Laid-Open No. 8-299796 and the catalyst described in International Publication No. WO 2011/048976.

Specific examples of the preferable catalyst include the catalysts used in the examples and the comparative examples, for example, Example 2 and Comparative Example 3, of International Publication No. WO 2011/048976. In addition, a more preferable atomic ratio (copper/zinc) of the copper atoms to the zinc atoms in the catalyst is within a range of 2.1 to 3.0. A methanol synthesis catalyst containing 3 to 20 mass% of alumina in addition to having the above-described atomic ratio is still more preferable. Such a catalyst is not particularly limited as described above and can be prepared by, for example, the method described in International Publication No. WO 2011/048976. More specifically, the catalyst can be prepared by a production method having a step of mixing an aqueous solution containing copper, an aqueous solution containing zinc and an alkali aqueous solution to generate a precipitate containing copper and zinc, a step of mixing the obtained precipitate and an alumina hydrate having a pseudo-boehmite structure to obtain a mixture and a step of molding the obtained mixture such that the density reaches 2.0 to 3.0 g/mL. Here, examples of the molding method include tableting, extrusion molding and tumbling granulation. However, the catalyst that is used in the present embodiment is not limited to the above-described catalysts and catalysts prepared by the above-described preparation methods and may be other catalysts having an equivalent methanol synthesis activity.

[Step (D)]

The step (D) in the method for producing methanol of the present embodiment is a step (D) of supplying the residual portion 7b of the reformed gas to a shift reaction unit 50 to be subjected to a shift reaction to obtain a shift reaction gas 10 as shown in Figures 1 to 4. The unreacted gas 9 may be jointly supplied with the residual portion 7b of the reformed gas to the shift reaction unit 50 as shown in Figures 3 and 4. The shift reaction is a reaction by which carbon monoxide in the reformed gas is reacted with steam to generate a shift reaction gas mainly containing water and carbon dioxide.

The shift reaction gas is a gas that is obtained by the step (D) and contains water and carbon dioxide that are generated by the reaction between carbon monoxide and hydrogen.

The shift reaction is preferably performed in the presence of a catalyst. As the catalyst, a known conventional catalyst can be used. The catalyst is not particularly limited, and examples thereof include transition metal oxides or platinum. The transition metal oxides are not particularly limited, and examples thereof include iron oxide ($Fe_3O_4$).

The residual portion of the reformed gas in the step (D) means, in the reformed gas that is obtained by the step (A), a molar flow rate of the reformed gas excluding the reformed gas that is used in the step (C). The molar flow rate of the reformed gas that is used in the step (D) is preferably 5 to 50 mol% and more preferably 10 to 40 mol% based on the molar flow rate of the reformed gas that is used in the step (A). When the molar flow rate of the reformed gas that is used in the step (D) is 5 mol% or more, there is a tendency that the reduction of the amount of carbon dioxide exhausted is superior, and, when the molar flow rate is 50 mol% or less, there is a tendency that the amount of methanol produced is superior.

The reaction temperature of the shift reaction is preferably 400°C to 700°C, and the reaction pressure is preferably 0.5 to 2.0 MPaG.

In the shift reaction, steam that is supplied from the outside of the system may be mixed into the shift reaction unit.

The carbon monoxide concentration in the shift reaction gas (10) is preferably 0.01 to 1.0 mol% and more preferably 0.01 to 0.1 mol%. In such a case, there is a tendency that it is possible to reduce the amount of carbon dioxide that is exhausted to the outside of the system by the combustion of carbon monoxide and it is possible to further decrease the amount of carbon dioxide exhausted per the amount of methanol produced.

[Step (E)]

The step (E) in the method for producing methanol of the present embodiment is a step of supplying the shift reaction gas 10 to a carbon dioxide separation unit 60 to separate carbon dioxide and obtain a carbon dioxide-rich gas 11 and the carbon dioxide separation unit off-gas 12 as shown in Figures 1 to 4.

As a method for separating carbon dioxide, a known convention method can be used. The method for separating carbon dioxide is not particularly limited, and examples thereof include a physical absorption method, a physical adsorption method, a chemical absorption method, a chemical adsorption method, a membrane separation method, a cryogenic separation method and an electroadsorption method.

In addition, there are no cases where the carbon dioxide-rich gas separated and recovered by the step (E) is exhausted in the atmosphere. This makes it possible to reduce the amount of carbon dioxide exhausted. A method for treating such a separated and recovered carbon dioxide-rich gas is not particularly limited, and examples thereof include burying in the ground by a CCS method, selling as dry ice or an industrial carbon dioxide gas and using as a raw material of chemical products.

In addition, the carbon dioxide separation unit off-gas that is obtained in the step (E) mainly contains hydrogen and thus may be mixed with the hydrocarbon-containing gas 1 and/or the reformed gas 5 as a hydrogen-containing gas, may be used as a heat source of the step (A) through the line 14 or may be used as a heat source of the boiler through a line 13.

In the step (E), based on the molar flow rate of carbon dioxide that is contained in the shift reaction gas 10 being 100 mol%, the molar flow rate of carbon dioxide that is contained in the carbon dioxide-rich gas is preferably 80 to 100 mol%, more preferably 90 to 100 mol% and still more preferably 95 to 99.9 mol%. In such a case, there is a tendency that the reduction of carbon dioxide exhaustion is superior.

In the step (E), there are cases where stream is required at the time of absorbing or adsorbing and separating carbon dioxide, and, at that time, the steam recovered in the step (A) or steam that is generated in the step (C) may be used.

In addition, the heat recovered in the step (A) may be used in the step (E) or a renewable energy may be used in a form of being externally supplied.

[Step (F)]

The method for producing methanol of the present embodiment preferably further includes a step (F). The step (F) is a step of supplying the hydrocarbon-containing gas 1 to the desulfurization unit 20 to obtain a sulfur-removed gas 4 from which the sulfur content has been removed as shown in Figures 2 to 4. In a case where the hydrocarbon-containing gas is a fossil fuel gas, since a sulfur content is contained, the step (F) is particularly required. That is, since a sulfur compound acts as a catalyst poison for the catalyst that is used in the step (A) or the step (C), in a case where the hydrocarbon-containing gas contains a sulfur content, it is preferable to remove the sulfur content in advance by the step (F). As a desulfurization method, a known conventional method can be used. The desulfurization method is not particularly limited, and examples thereof include dry methods in which an adsorbent or a catalyst is used and wet methods in which an amine-based or other absorption liquid is used. In the case of using a dry method, the operation temperature can be set to 0°C to 400°C, which cannot be said for sure depending on the kind of a sulfur compound that is a removal target or the kind of a catalyst that is employed.

[Step (G)]

The method for producing methanol of the present embodiment preferably further includes a step (G). The step (G) is a step of obtaining purified methanol from the methanol 8 while the detail thereof is not shown in Figures 1 to 4.

As a method for obtaining purified methanol in the step (G), a known conventional method can be used. As the method for obtaining purified methanol, for example, a distillation column including a reboiler and a condenser can be used while not particularly limited. In that case, high-purity methanol can be obtained from the bottom or middle of the column by distilling methanol.

In the step (G), the steam recovered in the step (A) or steam that is generated in the step (C) may be used. The use of steam makes it possible to absorb or adsorb a fluid that is exhausted from the top of the column in the case of diluting methanol using, for example, the distillation column. This makes it possible to further reduce the amount of carbon dioxide exhausted.

In the step (G), it is also possible to use the heat recovered in the step (A). Such heat can be used as heat that becomes necessary in the reboiler in the case of diluting methanol using, for example, the distillation column while not particularly limited. This makes it possible to further reduce the amount of carbon dioxide exhausted. In addition, a renewable energy may be used as the heat in a form of being externally supplied.

[Step (H)]

The method for producing methanol of the present embodiment preferably further includes a step (H). The detail of the step (H) is a step of supplying the carbon dioxide separation unit off-gas 12 to a methane separation unit 70 to separate methane from the carbon dioxide separation unit off-gas 12 and obtain a methane-rich gas 15 and the methane separation unit off-gas 16 as shown in Figure 4.

As a method for separating methane in the step (H), a known conventional method can be used. The method for separating methane is not particularly limited, and examples thereof include a physical adsorption method and a membrane separation method.

Regarding the methane separation efficiency, based on the molar flow rate of methane that is contained in the carbon dioxide separation unit off-gas being 100 mol%, the molar flow rate of methane that is contained in the methane-rich gas is preferably 50 to 99 mol%, more preferably 70 to 99 mol% and still more preferably 90 to 99 mol%. In such a case, there is a tendency that it is possible to reduce the amount of carbon dioxide that is exhausted to the outside of the system by the combustion of methane and it is possible to further decrease the amount of carbon dioxide exhausted per the amount of methanol produced.

The methane separation unit off-gas may be used as a heat source of the step (A) and/or a heat source of the boiler.

In addition, the methane separation unit off-gas 16 mainly contains hydrogen and thus may be mixed with the hydrocarbon-containing gas 1 and/or the reformed gas 5 as a hydrogen-containing gas, may be used as a heat source of the step (A) through the line 14 and may be used as a heat source of the boiler through a line 13.

In addition, the methane-rich gas 15 may be supplied to the reforming unit 30 through a line 2 as shown in Figure 4.

[Other Steps]

The method for producing methanol of the present embodiment may include other steps as necessary aside from each of the above-described steps.

The other steps are not particularly limited, and the method for producing methanol may have, for example, a methane-recovering step of mixing the methane-rich gas obtained in the step (H) with a hydrocarbon-containing gas.

[Apparatus for Producing Methanol]

An apparatus for producing methanol of the present embodiment is an apparatus for performing the above-described method for producing methanol, and examples thereof include apparatuses shown by the schematic views of Figures 1 to 4.

The apparatus for producing methanol of the present embodiment is an apparatus for producing methanol including the reforming unit 30, the methanol synthesis unit 40, the shift reaction unit 50 and the carbon dioxide separation unit 60 as shown in Figure 1, in which the carbon dioxide separation unit off-gas 12 that is obtained from the carbon dioxide separation unit 60 is used as a fuel of the reforming unit 30 and/or the boiler.

The reforming unit 30 includes a reforming reactor and may include other devices as necessary. In the reforming reactor, the hydrocarbon-containing gas and steam are reacted at a predetermined temperature to generate a reformed gas containing hydrogen ($H_2$), carbon monoxide (CO) and carbon dioxide ($CO_2$) as main components, which is used for the synthesis of methanol.

The methanol synthesis unit 40 includes a methanol synthesis reactor in which the reformed gas is reacted in the presence of a catalyst to generate methanol and the unreacted gas and may include other devices as necessary.

The shift reaction unit 50 includes a shift reactor and may include other devices as necessary. In the shift reactor, the reformed gas is subjected to a shift reaction to generate the shift reaction gas.

The carbon dioxide separation unit 60 includes a carbon dioxide separator that separates carbon dioxide from the shift reaction gas to generate the carbon dioxide-rich gas and the carbon dioxide separation unit off-gas and may include other devices as necessary.

In addition, the apparatus for producing methanol of the present embodiment preferably further includes the desulfurization unit 20 as shown in Figure 2.

The desulfurization unit 20 includes a desulfurizer that remove the sulfur content from the hydrocarbon-containing gas and may include other devices as necessary.

In the apparatus for producing methanol of the present embodiment, it is preferable that a line 9 through which the unreacted gas flows is connected to the shift reaction unit 50 as shown in Figures 3 and 4. In such a case, it is possible to further reduce the amount of carbon dioxide exhausted.

The apparatus for producing methanol of the present embodiment preferably further includes the methane separation unit 70 that separates methane from the carbon dioxide separation unit off-gas as shown in Figure 4. In such a case, it is possible to further reduce the amount of carbon dioxide exhausted.

The methane separation unit 70 includes a methane separator that separates methane from the carbon dioxide separation unit off-gas to generate the methane-rich gas and the methane separation unit off-gas and may include other devices as necessary.

[Examples]

Hereinafter, a method for producing methanol and an apparatus for producing methanol of the present invention will be described with examples and comparative examples, but the present invention is not limited thereto.

As a catalyst that was used for methanol synthesis, any of a catalyst prepared by the method described in Example 1 of Japanese Patent Publication No. 51-44715B (methanol synthesis catalyst A), a catalyst prepared by the method described in Example 1 of Japanese Patent Laid-Open No. 8-299796 (methanol synthesis catalyst B), a catalyst prepared by the method described in Example 3 of International Publication No. WO 2011/048976 (methanol synthesis catalyst C) or a catalyst prepared by the method described in Comparative Example 4 of Japanese Patent Laid-Open No. 8-299796 (methanol synthesis catalyst D) was used. Regarding the amount of the catalyst used in each of the examples and the comparative examples below, the amounts were all set to be the same as each other.

[Example 1]

In Example 1, a production apparatus shown in Figure 2 was used. Each condition was set as shown in Table 1. That is, a steam reforming reaction was performed using shale gas ($CH_4$: 94.3 mol%, $C_2H_6$: 2.7 mol%, $C_3H_8$: 0.6 mol%, $C_4H_{10}$: 0.2 mol%, $C_5H_{12}$: 0.2 mol%, $CO_2$: 0.5 mol%, $N_2$: 1.5 mol%) as a raw material gas, and then synthesis of methanol was performed using a synthesis gas that was generated by the steam reforming reaction. As the catalyst in a methanol synthesis reactor in a methanol synthesis unit, the methanol synthesis catalyst C was used. In addition, the temperature and pressure of each line were set to become values in Table 1. As the methanol synthesis reactor, a multi-tubular heat exchange-type reactor was used. Regarding set conditions, the pressure of a fluid that came into contact with the catalyst in the reactor was set to 10.0 MPaG, the shell pressure was set to 4.0 MPaG, and the temperature was somewhere between 200°C and 234°C. In addition, the reaction pressure in a reformer was 1.9 MPaG, and the temperature was 860°C. In addition, regarding the allocation of the molar flow rates in a line 7a and a line 7b, a distribution ratio was set so as to be capable of meeting the right amounts of heat required for heating in a reforming unit.

In addition, while not shown in Figure 2, a methanol 8 that was obtained from a methanol synthesis unit 40 was cooled to 45°C, which is the dew point of methanol or lower, to accelerate the condensation of methanol.

In addition, material balances regarding Example 1 are shown in Table 1. Line numbers shown in Figure 2 are shown in the vertical column of Table 1, and the temperature (°C), pressure (MPaG) and molar flow rate (kmol/h) of an object that circulates through each line are shown in the horizontal column.

[Table 1]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 8.6 | 0.0 | 0.0 | 0.0 | 0.0 | 2.5 | 0.3 | 202.0 | 0.9 | 1.2 | 0.0 | 0.0 |
| 3 | 40 | 2.5 | 3615.1 | 103.3 | 22.9 | 7.6 | 7.6 | 21.7 | 0.3 | 202.0 | 58.2 | 1.2 | 0.0 | 0.0 |
| 4 | 400 | 2.3 | 3615.1 | 103.3 | 22.9 | 7.6 | 7.6 | 21.7 | 0.3 | 202.0 | 58.2 | 1.2 | 0.0 | 0.0 |
| 5 | 520 | 2.0 | 577.6 | 0.0 | 0.0 | 0.0 | 0.0 | 1194.4 | 2209.2 | 11316.9 | 58.2 | 7324.6 | 0.0 | 0.0 |
| 7a | 45 | 2.0 | 468.7 | 0.0 | 0.0 | 0.0 | 0.0 | 969.3 | 1792.8 | 9183.7 | 47.3 | 14.0 | 0.0 | 0.0 |
| 7b | 520 | 2.0 | 108.9 | 0.0 | 0.0 | 0.0 | 0.0 | 225.1 | 416.4 | 2133.2 | 11.0 | 1380.7 | 0.0 | 0.0 |
| 8 | 45 | 0.3 | 1.4 | 0.0 | 0.0 | 0.0 | 0.0 | 5.2 | 0.0 | 1.1 | 0.0 | 1200.1 | 2649.7 | 4.6 |
| 9 | 45 | 2.0 | 467.3 | 0.0 | 0.0 | 0.0 | 0.0 | 57.1 | 37.5 | 2951.3 | 47.2 | 3.6 | 1.9 | 0.7 |
| 10 | 45 | 1.8 | 108.9 | 0.0 | 0.0 | 0.0 | 0.0 | 637.3 | 4.3 | 2545.4 | 11.0 | 15.2 | 0.0 | 0.0 |
| 11 | 45 | 0.1 | 0.0 | 0.0 | 0.0 | 0.0 | 0.0 | 605.4 | 0.0 | 0.0 | 0.0 | 0.0 | 0.0 | 0.0 |
| 12 | 45 | 1.6 | 108.9 | 0.0 | 0.0 | 0.0 | 0.0 | 31.9 | 4.3 | 2545.4 | 11.0 | 15.2 | 0.0 | 0.0 |
| 13 | 45 | 1.6 | 25.3 | 0.0 | 0.0 | 0.0 | 0.0 | 7.4 | 1.0 | 592.5 | 2.6 | 3.5 | 0.0 | 0.0 |
| 14 | 45 | 1.6 | 74.9 | 0.0 | 0.0 | 0.0 | 0.0 | 21.9 | 2.9 | 1750.9 | 7.6 | 10.4 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Example 1, are shown in Table 9.

The amount of methanol produced (ton/day) in Example 1 in Table 9 is calculated by multiplying the molar flow rate (kmol/h) of methanol in a line 8 in Table 1 by the molar mass (g/mol) of methanol and 24 (h).

In addition, the amount of carbon dioxide exhausted (ton/day) in Example 1 in Table 9 is calculated by multiplying the total of the molar flow rates (kmol/h) of carbon atoms in lines 9, 13 and 14 in Table 1 by the molar mass (g/mol) of carbon dioxide and 24 (h).

[Example 2]

In Example 2, a production apparatus shown in Figure 3 was used. Synthesis of methanol was performed under the same conditions as in Example 1 except that the lines were changed as shown in Figure 3. Material balances were as shown in Table 2.

[Table 2]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 21.0 | 0.0 | 0.0 | 0.0 | 0.0 | 1.3 | 0.3 | 202.7 | 2.2 | 1.2 | 0.0 | 0.0 |
| 3 | 40 | 2.5 | 3627.5 | 103.3 | 22.9 | 7.6 | 7.6 | 20.5 | 0.3 | 202.7 | 59.5 | 1.2 | 0.0 | 0.0 |
| 4 | 400 | 2.3 | 3627.5 | 103.3 | 22.9 | 7.6 | 7.6 | 20.5 | 0.3 | 202.7 | 59.5 | 1.2 | 0.0 | 0.0 |
| 5 | 520 | 2.0 | 579.5 | 0.0 | 0.0 | 0.0 | 0.0 | 1197.5 | 2215.4 | 11353.6 | 59.5 | 7346.9 | 0.0 | 0.0 |
| 7a | 45 | 2.0 | 469.4 | 0.0 | 0.0 | 0.0 | 0.0 | 970.0 | 1794.5 | 9196.4 | 48.2 | 14.1 | 0.0 | 0.0 |
| 7b | 520 | 2.0 | 110.1 | 0.0 | 0.0 | 0.0 | 0.0 | 227.5 | 420.9 | 2157.2 | 11.3 | 1395.9 | 0.0 | 0.0 |
| 8 | 45 | 0.3 | 1.4 | 0.0 | 0.0 | 0.0 | 0.0 | 5.2 | 0.0 | 1.1 | 0.0 | 1200.8 | 2652.0 | 4.6 |
| 9 | 45 | 2.0 | 467.9 | 0.0 | 0.0 | 0.0 | 0.0 | 57.1 | 37.6 | 2958.6 | 48.2 | 3.7 | 1.9 | 0.7 |
| 10 | 45 | 1.8 | 578.0 | 0.0 | 0.0 | 0.0 | 0.0 | 735.3 | 7.9 | 5566.4 | 59.5 | 31.8 | 1.9 | 0.7 |
| 11 | 45 | 0.1 | 0.0 | 0.0 | 0.0 | 0.0 | 0.0 | 698.5 | 0.0 | 0.0 | 0.0 | 0.0 | 0.0 | 0.0 |
| 12 | 45 | 1.6 | 578.0 | 0.0 | 0.0 | 0.0 | 0.0 | 36.8 | 7.9 | 5566.4 | 59.5 | 31.8 | 1.9 | 0.7 |
| 13 | 45 | 1.6 | 52.3 | 0.0 | 0.0 | 0.0 | 0.0 | 3.3 | 0.7 | 503.4 | 5.4 | 2.9 | 0.2 | 0.1 |
| 14 | 45 | 1.6 | 504.7 | 0.0 | 0.0 | 0.0 | 0.0 | 32.1 | 6.9 | 4860.4 | 52.0 | 27.8 | 1.7 | 0.6 |

The amount of methanol produced (ton/day), the amount of carbon dioxide exhausted (ton/day) and the amount of carbon dioxide exhausted per unit methanol (ton-$CO_2$/ton-MeOH), which were obtained in Example 2, are shown in Table 9.

The amount of methanol produced (ton/day) in Example 2 in Table 9 is calculated by multiplying the molar flow rate (kmol/h) of methanol in a line 8 in Table 2 by the molar mass (g/mol) of methanol and 24 (h).

In addition, the amount of carbon dioxide exhausted (ton/day) in Example 2 in Table 9 is calculated by multiplying the total of the molar flow rates (kmol/h) of carbon atoms in lines 13 and 14 in Table 2 by the molar mass (g/mol) of carbon dioxide and 24 (h).

[Example 3]

In Example 3, a production apparatus shown in Figure 4 was used. Synthesis of methanol was performed under the same conditions as in Example 1 except that the lines were changed as shown in Figure 4 and the material balances were changed as shown in Table 3. Material balances were as shown in Table 3.

[Table 3]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 600.2 | 0.0 | 0.0 | 0.0 | 0.0 | 2.0 | 0.4 | 233.2 | 1.7 | 1.4 | 0.0 | 0.0 |
| 3 | 40 | 2.5 | 4206.6 | 103.3 | 22.9 | 7.6 | 7.6 | 21.1 | 0.4 | 233.2 | 59.1 | 1.4 | 0.0 | 0.0 |
| 4 | 400 | 2.3 | 4206.6 | 103.3 | 22.9 | 7.6 | 7.6 | 21.1 | 0.4 | 233.2 | 59.1 | 1.4 | 0.0 | 0.0 |
| 5 | 520 | 2.0 | 666.2 | 0.0 | 0.0 | 0.0 | 0.0 | 1369.3 | 2536.9 | 13032.7 | 59.1 | 8421.0 | 0.0 | 0.0 |
| 7a | 45 | 2.0 | 438.9 | 0.0 | 0.0 | 0.0 | 0.0 | 902.1 | 1671.3 | 8585.9 | 38.9 | 13.1 | 0.0 | 0.0 |
| 7b | 520 | 2.0 | 227.3 | 0.0 | 0.0 | 0.0 | 0.0 | 467.2 | 865.6 | 4446.7 | 20.2 | 2873.3 | 0.0 | 0.0 |
| 8 | 45 | 0.3 | 1.4 | 0.0 | 0.0 | 0.0 | 0.0 | 4.5 | 0.0 | 1.1 | 0.0 | 1141.5 | 2478.1 | 4.3 |
| 9 | 45 | 2.0 | 437.5 | 0.0 | 0.0 | 0.0 | 0.0 | 48.3 | 30.9 | 2756.1 | 38.9 | 3.4 | 1.7 | 0.6 |
| 10 | 45 | 1.8 | 664.8 | 0.0 | 0.0 | 00 | 0.0 | 1399.8 | 12.2 | 8087.1 | 59.1 | 46.8 | 1.7 | 0.6 |
| 11 | 45 | 0.3 | 0.0 | 0.0 | 0.0 | 0.0 | 0.0 | 1329.8 | 0.0 | 0.0 | 0.0 | 0.0 | 0.0 | 0.0 |
| 12 | 45 | 1.6 | 664.8 | 0.0 | 0.0 | 0.0 | 0.0 | 70.0 | 12.2 | 8087.1 | 59.1 | 46.8 | 1.7 | 0.6 |
| 13 | 45 | 1.6 | 5.4 | 0.0 | 0.0 | 0.0 | 0.0 | 5.7 | 1.0 | 658.6 | 4.8 | 3.8 | 0.1 | 0.1 |
| 14 | 45 | 1.6 | 59.1 | 0.0 | 0.0 | 0.0 | 0.0 | 62.3 | 10.9 | 7195.4 | 52.5 | 41.7 | 1.5 | 0.5 |
| 15 | 45 | 0.3 | 598.3 | 0.0 | 0.0 | 0.0 | 0.0 | 0.0 | 0.0 | 0.0 | 0.0 | 0.0 | 0.0 | 0.0 |
| 16 | 45 | 1.5 | 1.9 | 0.0 | 0.0 | 0.0 | 0.0 | 2.0 | 0.4 | 233.2 | 1.7 | 1.4 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Example 3, are shown in Table 9.

The amount of methanol produced (ton/day) in Example 3 in Table 9 is calculated by multiplying the molar flow rate (kmol/h) of methanol in a line 8 in Table 3 by the molar mass (g/mol) of methanol and 24 (h).

In addition, the amount of carbon dioxide exhausted (ton/day) in Example 3 in Table 9 is calculated by multiplying the total of the molar flow rates (kmol/h) of carbon atoms in lines 13 and 14 in Table 3 by the molar mass (g/mol) of carbon dioxide and 24 (h).

[Comparative Example 1]

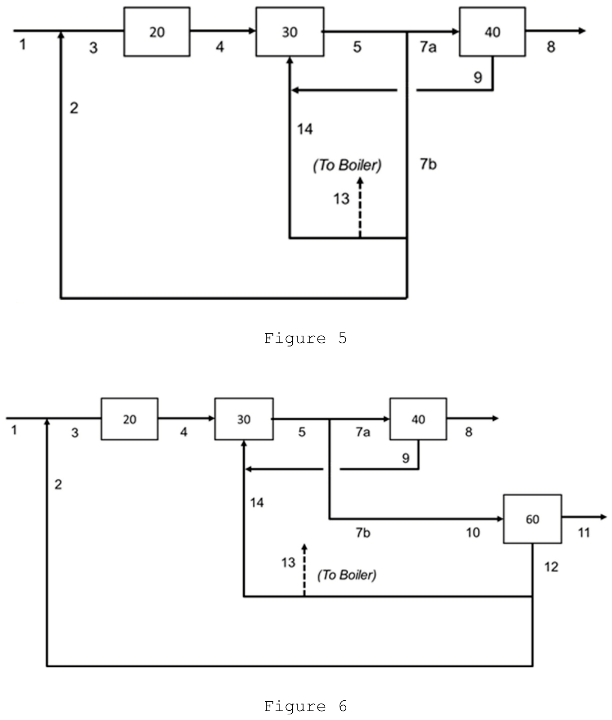

In Comparative Example 1, a production apparatus shown in Figure 5 was used. Synthesis of methanol was performed under the same conditions as in Example 1 except that the production apparatus shown in Figure 5 did not include the shift reaction unit and the carbon dioxide separation unit and the lines were changed as shown in Figure 5. Material balances were as shown in Table 4.

[Table 4]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 10.6 | 0.0 | 0.0 | 0.0 | 0.0 | 22.0 | 40.7 | 205.2 | 1.1 | 0.3 | 0.0 | 0.0 |
| 3 | 40 | 2.5 | 3617.0 | 103.3 | 22.9 | 7.6 | 7.6 | 41.2 | 40.7 | 205.2 | 58.4 | 0.3 | 0.0 | 0.0 |
| 4 | 400 | 2.3 | 3617.0 | 103.3 | 22.9 | 7.6 | 7.6 | 41.2 | 40.7 | 205.2 | 58.4 | 0.3 | 0.0 | 0.0 |
| 5 | 520 | 2.0 | 582.5 | 0.0 | 0.0 | 0.0 | 0.0 | 1215.4 | 2245.2 | 11312.6 | 58.4 | 17.4 | 0.0 | 0.0 |
| 7a | 45 | 2.0 | 466.6 | 0.0 | 0.0 | 0.0 | 0.0 | 973.5 | 1798.4 | 9061.4 | 46.8 | 13.9 | 0.0 | 0.0 |
| 7b | 520 | 2.0 | 115.9 | 0.0 | 0.0 | 0.0 | 0.0 | 241.9 | 446.8 | 2251.2 | 11.6 | 3.5 | 0.0 | 0.0 |
| 8 | 45 | 0.3 | 1.5 | 0.0 | 0.0 | 0.0 | 0.0 | 5.4 | 0.0 | 1.1 | 0.0 | 1203.4 | 2659.2 | 4.7 |
| 9 | 45 | 2.0 | 465.1 | 0.0 | 0.0 | 0.0 | 0.0 | 57.8 | 36.9 | 2806.8 | 46.8 | 3.6 | 1.8 | 0.7 |
| 13 | 45 | 1.6 | 24.9 | 0.0 | 0.0 | 0.0 | 0.0 | 51.9 | 95.9 | 483.0 | 2.5 | 0.7 | 0.0 | 0.0 |
| 14 | 45 | 1.6 | 80.5 | 0.0 | 0.0 | 0.0 | 0.0 | 167.9 | 310.2 | 1563.0 | 8.1 | 2.4 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Comparative Example 1, are shown in Table 9.

The amount of methanol produced (ton/day) in Comparative Example 1 in Table 9 is calculated by multiplying the molar flow rate (kmol/h) of methanol in a line 8 in Table 4 by the molar mass (g/mol) of methanol and 24 (h).

In addition, the amount of carbon dioxide exhausted (ton/day) in Comparative Example 1 in Table 9 is calculated by multiplying the total of the molar flow rates (kmol/h) of carbon atoms in lines 9, 13 and 14 in Table 4 by the molar mass (g/mol) of carbon dioxide and 24 (h).

[Comparative Example 2]

In Comparative Example 2, a production apparatus shown in Figure 6 was used. Synthesis of methanol was performed under the same conditions as in Example 1 except that the production apparatus shown in Figure 6 did not include the shift reaction unit and the lines were changed as shown in Figure 6. Material balances were as shown in Table 5.

[Table 5]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 10.5 | 0.0 | 0.0 | 0.0 | 0.0 | 1.1 | 40.3 | 204.1 | 1.1 | 1.3 | 0.0 | 0.0 |
| 3 | 40 | 2.5 | 3616.9 | 103.3 | 22.9 | 7.6 | 7.6 | 20.2 | 40.3 | 204.1 | 58.4 | 1.3 | 0.0 | 0.0 |
| 4 | 400 | 2.3 | 3616.9 | 103.3 | 22.9 | 7.6 | 7.6 | 20.2 | 40.3 | 204.1 | 58.4 | 1.3 | 0.0 | 0.0 |
| 5 | 520 | 2.0 | 582.8 | 0.0 | 0.0 | 0.0 | 0.0 | 1206.0 | 2232.8 | 11322.2 | 58.4 | 7320.5 | 0.0 | 0.0 |
| 7a | 45 | 2.0 | 472.9 | 0.0 | 0.0 | 0.0 | 0.0 | 978.6 | 1812.0 | 9187.9 | 47.4 | 14.1 | 0.0 | 0.0 |
| 7b | 520 | 2.0 | 109.9 | 0.0 | 0.0 | 0.0 | 0.0 | 227.3 | 420.9 | 2134.2 | 11.0 | 1379.9 | 0.0 | 0.0 |
| 8 | 45 | 0.3 | 1.5 | 0.0 | 0.0 | 0.0 | 0.0 | 5.4 | 0.0 | 1.1 | 0.0 | 1208.6 | 2677.1 | 4.7 |
| 9 | 45 | 2.0 | 471.5 | 0.0 | 0.0 | 0.0 | 0.0 | 58.0 | 37.5 | 2892.2 | 47.4 | 3.6 | 1.9 | 0.7 |
| 11 | 45 | 0.1 | 0.0 | 0.0 | 0.0 | 0.0 | 0.0 | 216.0 | 0.0 | 0.0 | 0.0 | 0.0 | 0.0 | 0.0 |
| 12 | 45 | 1.6 | 109.9 | 0.0 | 0.0 | 0.0 | 0.0 | 11.4 | 420.9 | 2134.2 | 11.0 | 13.3 | 0.0 | 0.0 |
| 13 | 45 | 1.6 | 24.9 | 0.0 | 0.0 | 0.0 | 0.0 | 2.6 | 95.4 | 483.6 | 2.5 | 3.0 | 0.0 | 0.0 |
| 14 | 45 | 1.6 | 74.5 | 0.0 | 0.0 | 0.0 | 0.0 | 7.7 | 285.3 | 1446.5 | 7.5 | 9.0 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Comparative Example 2, are shown in Table 9.

The amount of methanol produced (ton/day) in Comparative Example 2 in Table 9 is calculated by multiplying the molar flow rate (kmol/h) of methanol in a line 8 in Table 5 by the molar mass (g/mol) of methanol and 24 (h).

In addition, the amount of carbon dioxide exhausted (ton/day) in Comparative Example 2 in Table 9 is calculated by multiplying the total of the molar flow rates (kmol/h) of carbon atoms in lines 9, 13 and 14 in Table 5 by the molar mass (g/mol) of carbon dioxide and 24 (h).

[Comparative Example 3]

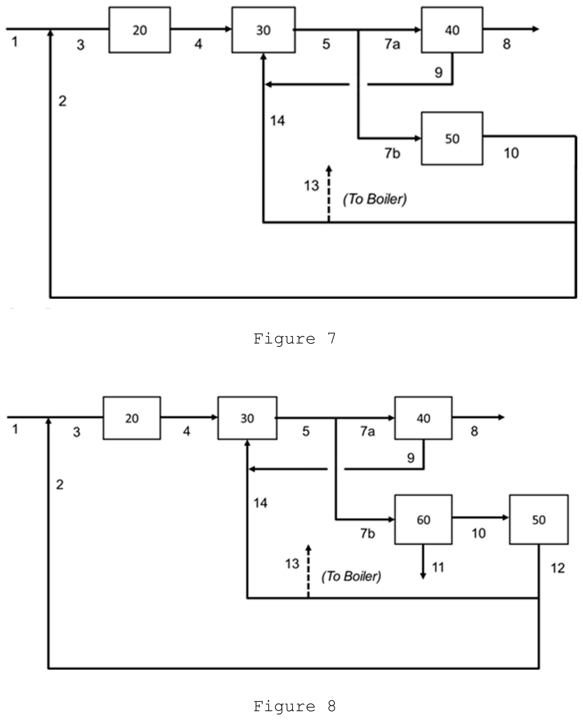

In Comparative Example 3, a production apparatus shown in Figure 7 was used. Synthesis of methanol was performed under the same conditions as in Example 1 except that the production apparatus shown in Figure 7 did not include the carbon dioxide separation unit and the lines were changed as shown in Figure 7. Material balances were as shown in Table 6.

[Table 6]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 8.7 | 0.0 | 0.0 | 0.0 | 0.0 | 52.0 | 0.3 | 204.6 | 0.9 | 1.2 | 0.0 | 0.0 |
| 3 | 40 | 2.5 | 3615.1 | 103.3 | 22.9 | 7.6 | 7.6 | 71.1 | 0.3 | 204.6 | 58.2 | 1.2 | 0.0 | 0.0 |
| 4 | 400 | 2.3 | 3615.1 | 103.3 | 22.9 | 7.6 | 7.6 | 71.1 | 0.3 | 204.6 | 58.2 | 1.2 | 0.0 | 0.0 |
| 5 | 520 | 2.0 | 576.8 | 0.0 | 0.0 | 0.0 | 0.0 | 1216.5 | 2237.5 | 11295.1 | 58.2 | 7351.3 | 0.0 | 0.0 |
| 7a | 45 | 2.0 | 448.7 | 0.0 | 0.0 | 0.0 | 0.0 | 946.4 | 1740.8 | 8787.6 | 45.3 | 13.5 | 0.0 | 0.0 |
| 7b | 520 | 2.0 | 128.0 | 0.0 | 0.0 | 0.0 | 0.0 | 270.1 | 496.7 | 2507.5 | 12.9 | 1632.0 | 0.0 | 0.0 |
| 8 | 45 | 0.3 | 1.5 | 0.0 | 0.0 | 0.0 | 0.0 | 4.7 | 0.0 | 1.1 | 0.0 | 1186.6 | 2593.3 | 4.6 |
| 9 | 45 | 2.0 | 447.2 | 0.0 | 0.0 | 0.0 | 0.0 | 47.9 | 29.3 | 2682.0 | 45.3 | 3.4 | 1.6 | 0.6 |
| 10 | 45 | 1.8 | 128.0 | 0.0 | 0.0 | 0.0 | 0.0 | 761.7 | 5.0 | 2999.2 | 12.9 | 17.9 | 0.0 | 0.0 |
| 13 | 45 | 1.6 | 25.3 | 0.0 | 0.0 | 0.0 | 0.0 | 150.5 | 1.0 | 592.6 | 2.6 | 3.5 | 0.0 | 0.0 |
| 14 | 45 | 1.6 | 94.0 | 0.0 | 0.0 | 0.0 | 0.0 | 559.3 | 3.7 | 2202.0 | 9.5 | 13.1 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Comparative Example 3, are shown in Table 9.

The amount of methanol produced (ton/day) in Comparative Example 3 in Table 9 is calculated by multiplying the molar flow rate (kmol/h) of methanol in a line 8 in Table 6 by the molar mass (g/mol) of methanol and 24 (h).

In addition, the amount of carbon dioxide exhausted (ton/day) in Comparative Example 3 in Table 9 is calculated by multiplying the total of the molar flow rates (kmol/h) of carbon atoms in lines 9, 13 and 14 in Table 6 by the molar mass (g/mol) of carbon dioxide and 24 (h).

[Comparative Example 4]

In Comparative Example 4, a production apparatus shown in Figure 8 was used. Synthesis of methanol was performed under the same conditions as in Example 1 except that the production apparatus shown in Figure 8 had the carbon dioxide separation unit disposed upstream of the shift reaction unit and the lines were changed as shown in Figure 8. Material balances were as shown in Table 7.

[Table 7]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | N2 | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 8.7 | 0.0 | 0.0 | 0.0 | 0.0 | 34.3 | 0.2 | 203.7 | 0.9 | 1.1 | 0.0 | 0.0 |
| 3 | 40 | 2.5 | 3615.1 | 103.3 | 22.9 | 7.6 | 7.6 | 53.4 | 0.2 | 203.7 | 58.2 | 1.1 | 0.0 | 0.0 |
| 4 | 400 | 2.3 | 3615.1 | 103.3 | 22.9 | 7.6 | 7.6 | 53.4 | 0.2 | 203.7 | 58.2 | 1.1 | 0.0 | 0.0 |
| 5 | 520 | 2.0 | 577.1 | 0.0 | 0.0 | 0.0 | 0.0 | 1208.5 | 2227.3 | 11302.8 | 58.2 | 17.3 | 0.0 | 0.0 |
| 7a | 45 | 2.0 | 454.5 | 0.0 | 0.0 | 0.0 | 0.0 | 972.8 | 1793.0 | 9098.8 | 46.9 | 13.9 | 0.0 | 0.0 |
| 7b | 520 | 2.0 | 112.5 | 0.0 | 0.0 | 0.0 | 0.0 | 235.7 | 434.3 | 2204.1 | 11.4 | 3.4 | 0.0 | 0.0 |
| 8 | 45 | 0.3 | 1.5 | 0.0 | 0.0 | 0.0 | 0.0 | 5.4 | 0.0 | 1.1 | 0.0 | 1203.1 | 2653.4 | 4.6 |
| 9 | 45 | 2.0 | 463.1 | 0.0 | 0.0 | 0.0 | 0.0 | 57.5 | 37.0 | 2856.0 | 46.9 | 3.6 | 1.8 | 0.7 |
| 10 | 45 | 1.8 | 112.5 | 0.0 | 0.0 | 0.0 | 0.0 | 11.8 | 434.3 | 2204.1 | 11.4 | 3.4 | 0.0 | 0.0 |
| 11 | 45 | 0.1 | 0.0 | 0.0 | 0.0 | 0.0 | 0.0 | 223.9 | 0.0 | 0.0 | 0.0 | 0.0 | 0.0 | 0.0 |
| 12 | 45 | 1.6 | 112.5 | 0.0 | 0.0 | 0.0 | 0.0 | 443.3 | 2.8 | 2635.6 | 11.4 | 6.2 | 0.0 | 0.0 |
| 13 | 45 | 1.6 | 21.3 | 0.0 | 0.0 | 0.0 | 0.0 | 84.0 | 0.5 | 499.3 | 2.2 | 2.8 | 0.0 | 0.0 |
| 14 | 45 | 1.6 | 82.5 | 0.0 | 0.0 | 0.0 | 0.0 | 325.1 | 2.0 | 1932.7 | 8.3 | 10.8 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Comparative Example 4, are shown in Table 9.

The amount of methanol produced (ton/day) in Comparative Example 4 in Table 9 is calculated by multiplying the molar flow rate (kmol/h) of methanol in a line 8 in Table 7 by the molar mass (g/mol) of methanol and 24 (h).

In addition, the amount of carbon dioxide exhausted (ton/day) in Comparative Example 4 in Table 9 is calculated by multiplying the total of the molar flow rates (kmol/h) of carbon atoms in lines 9, 13 and 14 in Table 7 by the molar mass (g/mol) of carbon dioxide and 24 (h).

[Comparative Example 5]

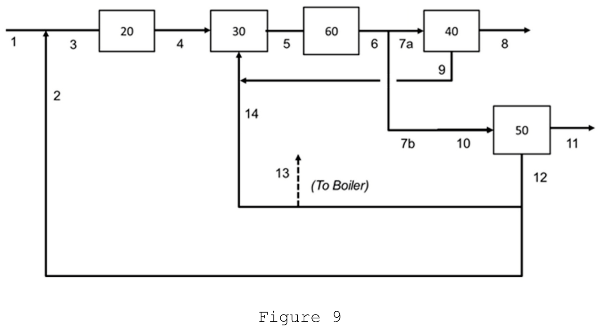

In Comparative Example 5, a production apparatus shown in Figure 9 was used. Synthesis of methanol was performed under the same conditions as in Example 1 except that the production apparatus shown in Figure 9 had the shift reaction unit disposed downstream of a line 5 and upstream of a line 6 and the lines were changed as shown in Figure 9. Material balances were as shown in Table 8.

In Comparative Example 5, in consideration of the thermal balance of the reforming unit, for example, the molar flow rate of a carbon raw material that flowed into the methanol synthesis unit became larger than in Example 1, and an amount of methanol produced equivalent to that in Example 1 was obtained in spite of the fact that the carbon yield failed to meet 96%.

[Table 8]

| Line number | Temperature [°C] | Pressure [MPaG] | Molar flow rate [kmol/h] | | | | | | | | | | | |
|---|---|---|---|---|---|---|---|---|---|---|---|---|---|---|
| | | | $CH_4$ | $C_2H_6$ | $C_3H_8$ | $C_4H_{10}$ | $C_5H_{12}$ | $CO_2$ | CO | $H_2$ | $N_2$ | $H_2O$ | $CH_3OH$ | Others |
| 1 | 30 | 2.0 | 3606.4 | 103.3 | 22.9 | 7.6 | 7.6 | 19.1 | 0.0 | 0.0 | 57.4 | 0.0 | 0.0 | 0.0 |
| 2 | 45 | 2.5 | 8.6 | 0.0 | 0.0 | 0.0 | 0.0 | 2.5 | 0.3 | 202.0 | 0.9 | 1.6 | 0.0 | 0.0 |
| 3 | 40 | 2.5 | 3615.1 | 103.3 | 22.9 | 7.6 | 7.6 | 21.7 | 0.3 | 202.0 | 58.2 | 1.6 | 0.0 | 0.0 |
| 4 | 400 | 2.3 | 3615.1 | 103.3 | 22.9 | 7.6 | 7.6 | 21.7 | 0.3 | 202.0 | 58.2 | 1.6 | 0.0 | 0.0 |
| 5 | 520 | 2.0 | 577.6 | 0.0 | 0.0 | 0.0 | 0.0 | 1194.4 | 2209.2 | 11317.1 | 58.2 | 7324.9 | 0.0 | 0.0 |
| 6 | 45 | 1.8 | 577.6 | 0.0 | 0.0 | 0.0 | 0.0 | 3381.0 | 22.7 | 13503.6 | 58.2 | 105.6 | 0.0 | 0.0 |
| 7a | 520 | 2.0 | 483.4 | 0.0 | 0.0 | 0.0 | 0.0 | 2829.9 | 19.0 | 11302.5 | 48.7 | 88.4 | 0.0 | 0.0 |
| 7b | 45 | 2.0 | 94.1 | 0.0 | 0.0 | 0.0 | 0.0 | 551.1 | 3.7 | 2201.1 | 9.5 | 17.2 | 0.0 | 0.0 |
| 8 | 45 | 0.3 | 1.2 | 0.0 | 0.0 | 0.0 | 0.0 | 11.0 | 0.0 | 1.0 | 0.0 | 3037.4 | 2641.8 | 4.7 |
| 9 | 45 | 2.0 | 482.3 | 0.0 | 0.0 | 0.0 | 0.0 | 148.5 | 34.6 | 3321.6 | 48.7 | 4.1 | 2.3 | 0.5 |
| 11 | 45 | 0.1 | 0.0 | 0.0 | 0.0 | 0.0 | 0.0 | 523.5 | 0.0 | 0.0 | 0.0 | 0.0 | 0.0 | 0.0 |
| 12 | 45 | 1.6 | 94.1 | 0.0 | 0.0 | 0.0 | 0.0 | 27.6 | 3.7 | 2201.1 | 9.5 | 17.2 | 0.0 | 0.0 |
| 13 | 45 | 1.6 | 25.3 | 0.0 | 0.0 | 0.0 | 0.0 | 7.4 | 1.0 | 592.5 | 2.6 | 4.6 | 0.0 | 0.0 |
| 14 | 45 | 1.6 | 60.2 | 0.0 | 0.0 | 0.0 | 0.0 | 17.6 | 2.4 | 1406.6 | 6.1 | 11.0 | 0.0 | 0.0 |

The amount of methanol produced (ton/day), the amount of carbon dioxide exhausted (ton/day) and the amount of carbon dioxide exhausted per unit methanol (ton-$CO_2$/ton-MeOH), which were obtained in Comparative Example 5, are shown in Table 9.

The amount of methanol produced (ton/day) in Comparative Example 5 in Table 9 is calculated by multiplying the molar flow rate (kmol/h) of methanol in a line 8 in Table 8 by the molar mass (g/mol) of methanol and 24 (h).

In addition, the amount of carbon dioxide exhausted (ton/day) in Comparative Example 5 in Table 9 is calculated by multiplying the total of the molar flow rates (kmol/h) of carbon atoms in lines 9, 13 and 14 in Table 8 by the molar mass (g/mol) of carbon dioxide and 24 (h).

The results obtained from Examples 1 to 3 and Comparative Examples 1 to 5 are shown in Table 9 below. Here, in a case where the carbon yield is 95.0 mol% or more and the amount of carbon dioxide exhausted/the amount of methanol produced is 0.400 or less, the case is equivalent to the examples, and otherwise the case is equivalent to the comparative examples.

[Table 9]

| | Unit | Examples | | | Comparative Examples | | | | |
|---|---|---|---|---|---|---|---|---|---|
| | | 1 | 2 | 3 | 1 | 2 | 3 | 4 | 5 |
| Methanol synthesis pressure | MPaG | 10.0 | 10.0 | 10.0 | 10.0 | 10.0 | 10.3 | 10.0 | 10.0 |
| Circulation ratio | - | 4.0 | 4.0 | 4.0 | 4.0 | 4.0 | 4.0 | 4.0 | 4.0 |
| Shell pressure | MPaG | 4.0 | 4.0 | 4.0 | 4.0 | 4.0 | 4.0 | 4.0 | 4.0 |
| M value at methanol synthesis unit inlet | - | 1.4 | 1.4 | 1.4 | 1.4 | 1.4 | 1.4 | 1.4 | 1.3 |
| $H_2$/CO at methanol synthesis unit inlet | - | 5.1 | 5.1 | 5.1 | 5.0 | 5.1 | 5.0 | 5.1 | 594.9 |
| Amount of catalyst | % | 100 | 100 | 100 | 100 | 100 | 100 | 100 | 100 |
| Carbon yield | % | 95.9 | 95.9 | 96.3 | 95.9 | 95.9 | 96.5 | 95.9 | 92.7 |
| Amount of methanol produced | ton/day | 2038 | 2039 | 1906 | 2045 | 2059 | 1994 | 2040 | 2031 |
| Amount of $CO_2$ exhausted | ton/day | 736 | 636 | 154 | 1366 | 1119 | 1436 | 1135 | 826 |
| Amount of $CO_2$ exhausted/amount of methanol produced | ton/ton | 0.361 | 0.312 | 0.081 | 0.668 | 0.544 | 0.720 | 0.556 | 0.406 |

Here, the definition of each term in Table 9 is as described below.

<Methanol Synthesis Pressure>

This means the gas pressure at the inlet of the methanol synthesis reactor.

<Circulation Ratio>

The circulation ratio is the ratio in the methanol synthesis unit of the molar flow rate of a circulation gas to the molar flow rate of the reformed gas that is supplied to the methanol synthesis unit. Here, the circulation gas refers to a gas that is obtained by separating methanol and water from an outlet gas of the methanol synthesis reactor in the methanol synthesis unit and is mixed with the reformed gas that is externally supplied to the same unit in the same unit and supplied to the methanol synthesis reactor.

<Shell Pressure>

This means the pressure inside the shell side and outside the tube in the multi-tubular heat exchange-type reactor.

<M Value at Methanol Synthesis Unit Inlet>

This means the M value at the inlet of the methanol synthesis unit.

<$H_2/CO$ at Methanol Synthesis Unit Inlet>

This means the ratio of the number of moles of hydrogen to the number of moles of carbon monoxide at the inlet of the methanol synthesis unit.

<Amount of Catalyst>

This means the mass ratio of the amount of the catalyst in each of the examples and the comparative examples based on the amount of the catalyst used in the production apparatus of Example 1 being 100%.

<Carbon Yield>

This means the ratio of the molar flow rate of methanol that is contained in the outlet gas of the methanol synthesis unit to the total amount of the molar flow rate of the carbon monoxide and the molar flow rate of carbon dioxide that are contained in the reformed gas that is supplied to the methanol synthesis unit.

[Industrial Applicability]

The present invention is industrially applicable in methods for producing methanol and apparatuses for producing methanol.

[Reference Signs List]

20 ⋯ desulfurization unit, 30 ⋯ reforming unit, 40 ⋯ methanol synthesis unit, 50 ⋯ shift reaction unit, 60 ⋯ carbon dioxide separation unit, 70 ⋯ methane separation unit, 1, 2, 3, 4, 5, 6, 7a, 7b, 8, 9, 10, 11, 12, 13, 14, 15, 16 ⋯ line

**Claims**

1. A method for producing methanol, comprising:

   a step (A) of reforming a hydrocarbon-containing gas to obtain a reformed gas,
   a step (B) of mixing a hydrogen-containing gas with the hydrocarbon-containing gas and/or the reformed gas,
   a step (C) of reacting a portion of the reformed gas in the presence of a catalyst to obtain methanol and an unreacted gas,
   a step (D) of subjecting a residual portion of the reformed gas to a shift reaction to obtain a shift reaction gas, and
   a step (E) of separating carbon dioxide from the shift reaction gas to obtain a carbon dioxide-rich gas and a carbon dioxide separation unit off-gas,
   wherein at least a portion of the carbon dioxide separation unit off-gas is used as a fuel of the step (A) and/or a boiler.

2. The method for producing methanol according to claim 1, further comprising
   a step (F) of removing a sulfur content from the hydrocarbon-containing gas.

3. The method for producing methanol according to claim 1, further comprising
   a step (G) of obtaining purified methanol from the methanol.

4. The method for producing methanol according to claim 1,
   wherein a molar flow rate of the reformed gas that is used in the step (C) is 50 to 95 mol% based on a molar flow rate of the reformed gas that is obtained in the step (A).

5. The method for producing methanol according to claim 3,
   wherein steam that is generated in the step (C) is used in the step (G) and/or the step (E).

**6.** The method for producing methanol according to claim 3,
wherein steam recovered in the step (A) is used in the step (G) and/or the step (E).

**7.** The method for producing methanol according to claim 3,
wherein heat recovered in the step (A) is used as a heat source in at least any of the step (G) and the step (E).

**8.** The method for producing methanol according to claim 1,
wherein the step (D) is performed in the presence of a catalyst.

**9.** The method for producing methanol according to claim 1,
wherein a carbon monoxide concentration in the shift reaction gas is 0.01 to 1.0 mol%.

**10.** The method for producing methanol according to claim 1,
wherein a molar flow rate of carbon dioxide that is contained in the carbon dioxide-rich gas is 80 to 100 mol% based on a molar flow rate of carbon dioxide that is contained in the shift reaction gas being 100 mol%.

**11.** The method for producing methanol according to claim 1,
wherein at least a portion of the unreacted gas is supplied to a shift reaction unit and/or the boiler.

**12.** The method for producing methanol according to claim 1, further comprising

a step (H) of separating methane from the carbon dioxide separation unit off-gas to obtain a methane-rich gas and a methane separation unit off-gas,
wherein the methane separation unit off-gas is supplied to a fuel of the step (A) and/or the boiler.

**13.** The method for producing methanol according to claim 12,
wherein the carbon dioxide separation unit off-gas and/or the methane separation unit off-gas is mixed with the hydrocarbon-containing gas and/or the reformed gas.

**14.** The method for producing methanol according to claim 12,
wherein a molar flow rate of methane that is contained in the methane-rich gas is 50 to 99 mol% based on a molar flow rate of methane that is contained in the carbon dioxide separation unit off-gas being 100 mol%.

**15.** An apparatus for producing methanol, comprising:

a reforming unit;
a methanol synthesis unit;
a shift reaction unit; and
a carbon dioxide separation unit,
wherein a carbon dioxide separation unit off-gas that is obtained from the carbon dioxide separation unit is used as a fuel of the reforming unit and/or a boiler.

**16.** The apparatus for producing methanol according to claim 15, further comprising
a desulfurization unit.

**17.** The apparatus for producing methanol according to claim 15, further comprising
a methane separation unit that separates methane from the carbon dioxide separation unit off-gas.

Figure 1

Figure 2

Figure 3

Figure 4

Figure 5

Figure 6

Figure 7

Figure 8

Figure 9

EP 4 644 356 A1

## INTERNATIONAL SEARCH REPORT

| International application No. |
|---|
| **PCT/JP2023/046092** |

**A. CLASSIFICATION OF SUBJECT MATTER**

*C07C 29/151*(2006.01)i; *C01B 3/38*(2006.01)i; *C07B 61/00*(2006.01)i; *C07C 31/04*(2006.01)i
FI: C07C29/151; C07C31/04; C01B3/38; C07B61/00 300

According to International Patent Classification (IPC) or to both national classification and IPC

**B. FIELDS SEARCHED**

Minimum documentation searched (classification system followed by classification symbols)

C07C29/00; C01B3/00; C07C31/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2002-363578 A (BABCOCK-HITACHI KABUSHIKI KAISHA) 18 December 2002 (2002-12-18)<br>    claims 1-4, paragraphs [0009]-[0014] | 1-17 |
| Y | JP 2009-179804 A (MITSUBISHI HEAVY INDUSTRIES, LTD.) 13 August 2009 (2009-08-13)<br>    claims 1-4, paragraphs [0026], [0050], [0056], fig. 6 | 1-17 |
| Y | JP 2010-138051 A (TOYO ENGINEERING CORPORATION) 24 June 2010 (2010-06-24)<br>    claims 1-6, paragraphs [0020], [0024] | 1-17 |
| Y | JP 2004-315474 A (MITSUBISHI HEAVY INDUSTRIES, LTD.) 11 November 2004 (2004-11-11)<br>    claims 1-11, paragraph [0005] | 1-17 |
| A | JP 2000-063115 A (MITSUBISHI GAS CHEMICAL COMPANY, INC.) 29 February 2000 (2000-02-29)<br>    claims 1-7 | 1-17 |

☑ Further documents are listed in the continuation of Box C.       ☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 March 2024** | **19 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

35

## INTERNATIONAL SEARCH REPORT

| International application No. |
| --- |
| **PCT/JP2023/046092** |

**C. DOCUMENTS CONSIDERED TO BE RELEVANT**

| Category* | Citation of document, with indication, where appropriate, of the relevant passages | Relevant to claim No. |
| --- | --- | --- |
| A | JP 01-180841 A (MITSUBISHI GAS CHEMICAL COMPANY, INC.) 18 July 1989 (1989-07-18)<br>claims 1-3 | 1-17 |

Form PCT/ISA/210 (second sheet) (July 2022)

| Patent document cited in search report | | | Publication date (day/month/year) | Patent family member(s) | Publication date (day/month/year) |
|---|---|---|---|---|---|
| JP | 2002-363578 | A | 18 December 2002 | (Family: none) | |
| JP | 2009-179804 | A | 13 August 2009 | (Family: none) | |
| JP | 2010-138051 | A | 24 June 2010 | US 2010/0150810 A1 claims 1-6, paragraph [0037] EP 2196448 A1 | |
| JP | 2004-315474 | A | 11 November 2004 | (Family: none) | |
| JP | 2000-063115 | A | 29 February 2000 | (Family: none) | |
| JP | 01-180841 | A | 18 July 1989 | US 5063250 A claims 1-4 GB 2213817 A DE 3900653 A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3674261 A
- US 20170197894 A
- EP 3844135 A
- US 10160704 B
- JP 51044715 A
- JP 2695663 B
- JP 6035401 A
- JP 10272361 A
- JP 2001205089 A
- JP 8299796 A
- WO 2011048976 A
- JP 51044715 B